# EUROPEAN PATENT APPLICATION

(11) **EP 1 878 800 A1**
(43) Date of publication of application: **16.01.2008**
(21) Application number: 06745973.5
(22) Date of filing: 25.04.2006
(51) Int. Cl.: C12Q 1/02, A61K 45/00, A61P 3/04, C12N 15/09, C12Q 1/68

(54) **METHOD FOR EVALUATING COMPOUND USING BARLP AND SUBSTANCE FOR SUPPRESSING EATING AND BODY WEIGHT**

(30) Priority: 26.04.2005 JP 2005127772
(71) Applicant: BANYU PHARMACEUTICAL CO., LTD., Tokyo 102-8667 (JP)
(72) Inventor: NAMBU, Hirohide, Tsukuba Res. Inst. of Banyo, Tsukuba-shi, Ibaraki 3002611 (JP); OZAKI, Satoshi, Tsukuba Res. Inst. of Banyo, Tsukuba-shi, Ibaraki 3002611 (JP); TANAKA, Takeshi, Tsukuba Res. Inst. of Banyo, Tsukubashi, Ibaraki 3002611 (JP); OHTA, Hisashi, Tsukuba Research Inst. of Banyo, Tsukuba-shi, Ibaraki 3002611 (JP)
(74) Representative: Buchan, Gavin MacNicol
(86) International application number: PCT/JP2006/309122
(87) International publication number: WO 2006/115297

(57) **Abstract**

It is intended to provide a method for evaluating a compound which regulates eating or body weight **characterized by** comprising the steps of introducing a BARLP gene and preparing a cell expressing BARLP; bringing a test compound into contact with the cells and detecting a specific binding of the test compound to the BARLP and a method for evaluating a compound further comprising the step of evaluating a test compound using a non human genetically engineered animal in which the BARLP gene is inactivated. According to this invention, knowledge about a relationship between BARLP and biological functions is obtained and a method for evaluating a compound targeting BARLP and a BARLP ligand obtained by the evaluation can be provided.

## Description

### Technical Field

The present invention relates to a method for evaluating a compound using BARLP (hereinafter a BARLP gene is referred to as a "BARLP gene" or merely "BARLP", and a BARLP protein is referred to as a "BARLP protein" or merely "BARLP"). Further, the present invention relates to a substance that regulates body weight having an action of regulating the expression of BARLP.

### Background Art

Most hormones, neurotransmitters or bioactive substances that regulate body functions transmit signals to target cells via guanosine triphosphate-binding protein (hereinafter, referred to as "G protein")-coupled receptors (hereinafter referred to as GPCR) present on cell membranes, whereby their unique functions are exhibited. Such receptors have a seven membrane spanning structure in common and form the G protein-coupled receptor superfamily.

Several hundred different G protein-coupled receptors have already been isolated to date, but a large number of so-called orphan receptors whose ligands are still unknown also exist.

Isolation of these receptors and ligands and elucidation of their functions will lead to understanding of their physiological function in the body, and also permit screening of agonists or antagonists capable of controlling the function, and thus it is expected to contribute to the development of new pharmaceuticals.

BARLP (biogenic amine receptor-like protein: also referred to as GPR61) is GPCR cloned by Lee et al. (Non-patent document 1) and Cikos et al. (Non-patent document 2). A human BARLP gene contains a 1353 bp open reading frame and encodes 451 amino acids (Accession No. AF258342). Further, BARLP is expressed mainly in the brain, and in particular, it is strongly expressed in the cerebral cortex, occipital pole, frontal lobe, temporal lobe, amygdala, hippocampus and the like. Meanwhile, the expression thereof is also observed in the putamen, caudate nucleus, and the like, but not observed in the spinal cord, corpus callosum and the like.

Further, BARLP shows a high homology of 28 to 31% at the amino acid level with various biogenic amine receptors (for example, respective receptors for serotonin, histamine, adrenaline and dopamine) that function in the brain.

Based on these findings, it is considered that BARLP is one of the molecules associated with the regulation of higher brain function typified by perception, recognition, memory or mental function.
Non-patent document 1: Molec. Brain Res., vol. 86, pp. 13-22, 2001
Non-patent document 2: Biochem. Biophys. Acta., vol. 1521, pp. 66-72, 2001

### Disclosure of the Invention

However, the present situation is that there is no report showing a direct relationship between BARLP and a specific brain function.

The present invention has been made in view of the above-mentioned problem of the conventional technique, and has its object to obtain knowledge about a relationship between BARLP and a biological function including a specific brain function and to provide a method for evaluating a compound targeting BARLP, and a ligand for BARLP obtained by the evaluation.

The present inventors made intensive studies in order to achieve the above object, and as a result, they found that there is a certain relationship between the expression level of BARLP and the body weight or amount of food intake, and that it becomes possible to evaluate a compound targeting BARLP based on this relationship, and thus completed the present invention.

That is, the method for evaluating a compound of the present invention is a method for evaluating a compound that regulates eating or body weight, and is characterized by comprising the steps of: preparing a cell expressing BARLP by introducing a BARLP gene; bringing a test compound into contact with the cell; and detecting a specific binding of the test compound to the BARLP.

Further, the method for evaluating a compound of the present invention is a method for evaluating a compound that regulates eating or body weight, and is characterized by comprising the steps of: preparing a cell expressing BARLP by introducing a BARLP gene; bringing a test compound into contact with the cell; measuring the activity of an intracellular signal transducer induced by the contact; and comparing the above-mentioned activity with the activity of the intracellular signal transducer in the absence of contact with the test compound.

Further, the method for evaluating a compound of the present invention is a method for evaluating a compound that regulates eating or body weight, and is characterized by comprising the steps of: preparing a cell expressing BARLP by introducing a BARLP gene; bringing a test compound into contact with the cell; measuring the expression level of the BARLP or an intracellular signal transducer mediated by the BARLP; and selecting the test compound which increased or decreased the expression level of the BARLP in comparison with the case in the absence of contact with the test compound.

Further, the method for evaluating a compound of the present invention is characterized by further comprising the step of evaluating a test compound using a nonhuman genetically modified animal in which the BARLP gene has been inactivated. By going through such a step, it becomes possible to also evaluate the behavior of the test compound in vivo, which permits more effective evaluation of a compound.

Further, the method for evaluating a compound of the present invention is a method for evaluating a compound that regulates eating or body weight, and is characterized by comprising the steps of: administering a test compound to a nonhuman genetically modified animal in which a BARLP gene has been inactivated; and detecting a change in a phenotype of the nonhuman genetically modified animal caused by the administration and comparing a difference with a phenotype of a normal animal.

Further, the method for evaluating a compound of the present invention is a method for evaluating a compound that regulates eating or body weight, and is characterized by comprising the steps of: bringing a test compound into contact with BARLP; and detecting a change in the activity of BARLP caused by the contact.

By the method for evaluating a compound as described above, it becomes possible to evaluate a compound binding to BARLP (for example, an agonist or an antagonist), which permits measurement of the activity of a test compound and screening of a compound having a desired activity.

Further, the ligand of the present invention is characterized in that it is isolated by any of the above-mentioned methods for evaluating a compound. With the use of such a ligand, it becomes possible to regulate eating or body weight, which permits the provision of a drug effective in prevention or treatment of a disease associated with the regulation of eating or body weight.

Further, the substance that regulates eating or body weight of the present invention is characterized by having an action of regulating a function of BARLP. With the use of such a substance that regulates body weight, it becomes possible to regulate eating or body weight, which permits the provision of a drug effective in prevention or treatment of a disease associated with the regulation of eating or body weight.

That is, according to the present invention, there is a certain relationship between the expression level of BARLP and the body weight or amount of food intake, and it becomes possible to evaluate a compound targeting BARLP based on the relationship. Further, it becomes possible to provide the ligand for BARLP (for example, a substance that regulates body weight) obtained by the evaluation.

### Brief Description of the Drawings

Fig. 1 is a graph showing changes in body weight of BARLP (-/-) and WT mice.
Fig. 2 is a graph showing comparison of amounts of food intake of BARLP (-/-) and WT mice at 8 to 11 weeks of age.
Fig. 3 is a graph showing comparison of amounts of food intake of BARLP (-/-) and WT mice at 12 to 15 weeks of age.
Fig. 4 is a graph showing comparison of amounts of food intake of BARLP (-/-) and WT mice at 16 to 19 weeks of age.
Fig. 5 is a graph showing comparison of amounts of food intake of BARLP (-/-) and WT mice at 20 to 23 weeks of age.
Fig. 6 is a chart showing changes in locomotor activity of BARLP (-/-) and WT mice.
Fig. 7 is a graph showing comparison of rectal temperatures of BARLP (-/-) and WT mice at 17 weeks of age.
Fig. 8 is a graph showing comparison of rectal temperatures of BARLP (-/-) and WT mice at 19 weeks of age.

### Best Mode for Carrying Out the Invention

Hereinafter, a preferred embodiment of the present invention will be described in detail.

The term "BARLP gene" as used herein is not particularly limited in terms of species from which the gene is derived, and examples thereof include humans, monkeys, mice, rats, dogs and rabbits. In particular, because a subject to which a compound to be evaluated is administered is humans, it is preferably a human BARLP gene.

Further, in the BARLP gene according to the present invention, a gene in which one or more base are substituted, deleted, added or inserted is also included as long as it has a physiological function equivalent to BARLP and encodes a protein that functions as GPCR. Here, the gene is not particularly limited in terms of its sequence as long as it is a gene encoding such a protein. However, the homology is preferably 50% or more, more preferably 70% or more, further more preferably 80% or more, and particularly preferably 90% or more (for example, 91, 92, 93, 94, 95, 96, 97, 98, 99% or more).

Further, in the BARLP gene according to the present invention, a nucleic acid which is hybridized to the BARLP gene under stringent conditions is also included. Here, the phrase "which is hybridized under stringent conditions" means that two nucleic acid fragments are hybridized to each other under the hybridization conditions described in Molecular Cloning : A Laboratory Manual 2nd Edition, Cold Spring Harbor (1989) 9.47-9.62 and 11.45-11.61. To be more specific, for example, conditions in which washing is carried out at 50°C with 2.0 × SSC after hybridization is carried out at about 45°C with 6.0 × SSC can be exemplified. In order to select the stringency, the salt concentration in the washing step can be selected from about 2.0 × SSC at 50°C as a low stringency to about 0.2 × SSC at 50°C as a high stringency. Further, the temperature in the washing step can be raised from room temperature of about 22°C as a low stringency condition to about 65°C as a high stringency condition.

As described above, BARLP had been presumed to be a molecule associated with the regulation of higher brain function typified by perception, recognition, memory or mental function in the central nervous system, however, the present inventors found that BARLP is associated with the regulation of body weight and amount of food intake. That is, in a nonhuman mammal in which BARLP was deleted, although there was no change in the motor activity and body temperature, an increase in the body weight and amount of food intake was observed. This means that body weight and eating can be regulated by a substance (for example, an agonist, an antagonist or the like) that regulates the expression level or function of BARLP.

### (1) Evaluation of compound

A compound that acts on BARLP can be evaluated using a BARLP gene or protein. Examples of the method of detecting an action against BARLP include a method of detecting a specific binding of a test compound to the receptor, a method of detecting the expression level of a gene which is changed by the contact with a test compound and a method of detecting an activity of intracellular signal transduction mediated by BARLP induced by the contact. Hereinafter, these methods will be described in turn.

First, the method for evaluating a test compound by detecting a specific binding of a test compound to the receptor will be described.

The method for evaluating a compound of the present invention is characterized by comprising the steps of: preparing a cell expressing BARLP by introducing a BARLP gene; bringing a test compound into contact with the cell; and detecting a specific binding of the test compound to the BARLP.

Further, the second method for evaluating a compound of the present invention is characterized by comprising the steps of: preparing a cell expressing BARLP by introducing a BARLP gene; bringing a test compound into contact with the cell; measuring the activity of an intracellular signal transducer induced by the contact; and comparing the above-mentioned activity with the activity of the intracellular signal transducer in the absence of contact with the test compound.

The test compound is not particularly limited, and examples thereof include single compounds such as natural compounds, organic compounds, inorganic compounds, proteins and peptides, and expression products of compound libraries and gene libraries, cell extracts, cell culture supernatants, fermentation products of microorganisms, extracts of marine organisms, plant extracts, prokaryotic cell extracts, eukaryotic single cell extracts, animal cell extracts and the like. The above-mentioned test samples can be used by appropriately labeling if necessary. As the labeling, for example, radiolabeling, fluorescent labeling and the like can be exemplified. Further, in addition to the above test samples, a mixture obtained by mixing plural types of these test samples is also included.

Further, the cell expressing a BARLP gene can be prepared by a method known to a person skilled in the art, and a specific method is not particularly limited, and, for example, the following method can be employed. That is, it is prepared by cloning a BARLP gene or a nucleic acid consisting of a part thereof into an expression vector containing a suitable promoter and a transcriptional regulatory element, and introducing the vector with the cloned nucleic acid into a host cell. Here, the vector is not particularly limited as long as it can be used as an expression vector, and examples thereof include pCMV-Tag, pcDNA3.1, pBlueBacHis2, pCI-neo, pcDNAI, pMC1neo, pXT1, pSG5, pEF1/V5-HisB, pCR2.1, pET11, λgt11 and pCR3.1.

Subsequently, the expression vector into which the BARLP gene or the nucleic acid consisting of a part thereof is introduced is transfected into a host cell. Such a host cell is not particularly limited as long as it is commonly used in the expression of a gene, and may be any of an animal cell, an insect cell, a plant cell, a microorganism. Specific examples thereof include COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and specific examples thereof include electroporation, the calcium phosphate method, the DEAE-dextran method, the lipofection method and the gene gun method.

Subsequently, the thus prepared cell expressing BARLP is brought into contact with a test compound. The method of contact is not particularly limited, and for example, if BARLP is in a purified state, the contact can be carried out by adding a test sample to a purified preparation. Further, if BARLP is in a state of expressing in a cell (including on a cell membrane) or in a state of expressing in a cell extract, the contact can be carried out by adding a test sample to a cell culture solution or the cell extract, respectively. In the case where a test sample is a protein, for example, a vector containing a DNA encoding the protein is transfected into a cell expressing BARLP. Alternatively, the contact can also be carried out by adding the vector to a cell extract in which BARLP is expressed.

The binding of a receptor expressed on a cell surface to a test compound can be detected by, for example, a label attached to the bound compound (for example, detection of binding amount by radioactivity or fluorescence intensity), and other than this, it can be detected using signal transduction into the cell caused by binding of the test compound to the receptor on the cell membrane (for example, G protein activation, change in the concentration of Ca²⁺ or cAMP (FLIPR (fluorometric imaging plate reader) or the like can be employed), phospholipase C activation, pH change, or receptor internalization) as an indicator. Further, the expression level or activity of a molecule (also including BARLP) involved in a signal transduction induced by the above-mentioned signal transduction can also be used as an indicator. Here, in the case where the expression level is used as an indicator, the method of measuring the expression level is not particularly limited, and examples thereof include Northern blotting, Western blotting and a DNA chip. Here, the term "expression level" as used herein refers to the absolute amount or relative amount of a transcription product of a gene encoding a protein in the signal transduction pathway mediated by BARLP. In this case, in the gene, both DNA and mRNA are included. Further, in the case where the detection target for expression is a protein, the term "expression level" refers to the absolute amount or relative amount of a translation product of a protein in the signal transduction pathway mediated by BARLP. Further, in the case where the activity of a molecule involved in signal transduction is used as an indicator, the method of measuring the activity is not particularly limited, and a suitable method may be selected depending on the type of a molecule to be measured.

On the other hand, an isolated BARLP protein can also be used directly for evaluation of a compound. That is, it is a method comprising bringing a test compound into contact with BARLP and detecting a change in the activity of the BARLP protein caused by the contact.

The method of such contact is not particularly limited, and specific examples thereof include a method in which the contact is achieved by mixing in a solution such as a buffer solution (a phosphate buffer solution or the like), and a method in which a BARLP protein is immobilized on a membrane and bringing the protein into contact with a test compound on the membrane.

Subsequently, a change in the activity of BARLP caused by the contact is detected.

The method of measuring the activity of a protein may be appropriately selected depending on the nature of a protein to be used, and specific examples thereof include a method in which the binding activity of a ligand for BARLP is used as an indicator.

The method in which the binding activity of a ligand is used as an indicator is not particularly limited, and specific examples thereof include a method in which the binding activity is determined by measuring affinity of a test compound for a membrane on which BARLP is immobilized. The test compound used here may be labeled with a radioisotope or the like so as to facilitate the detection. Further, as the method of detecting the binding activity, a method in which a compound binding to BARLP in a manner competitive with a ligand labeled with a radioisotope is detected can be exemplified. In the case where such a method is used, the test compound does not need to be labeled.

As described above, in the case where as a result of detecting a compound by the method for evaluating a compound of the present invention, the binding activity of a ligand in the presence of a test compound has a lower value than the binding activity in the absence of the test compound (control), the test compound is judged to have an activity of inhibiting the binding between the BARLP according to the present invention and the ligand. Such compounds include compounds which have an activity of inducing signal transduction into a cell upon binding to the receptor (agonists), compounds which do not have such an activity (antagonists) and the like. An agonist has the same physiological activity as the ligand for the receptor and its analogs, while an antagonist inhibits the physiological activity of the ligand for the receptor and its analogs. Thus, such agonists and antagonists are useful as a pharmaceutical composition for treatment of a disease caused by abnormalities in signal transduction systems mediated by BARLP according to the present invention.

Further, by the method for evaluating a compound of the present invention, screening of a substance that promotes or inhibits intracellular signal transduction after binding of a test compound to BARLP. That is, by evaluating multiple test compounds by the above-mentioned method, a compound that functions as an agonist or an antagonist can be selected. If, as a result of such selection, in comparison with the change in intracellular signal transduction in the case where the ligand or its analog is allowed to act in the absence of the test compound, the change is suppressed, the test compound is judged to be a compound that inhibits the intracellular signal transduction after binding of the test compound to BARLP. On the contrary, if the test compound enhances intracellular signal transduction, the compound is judged to be a compound that promotes the intracellular signal transduction after binding of the test compound to BARLP. A compound selected by such a screening method is effective in suppression of obesity or leanness based on regulation of eating or body weight and is useful for prevention or treatment of such a pathologic condition.

Further, in the method for evaluating a compound of the present invention, in addition to the above-mentioned evaluation method, the step of evaluating a test compound using a nonhuman genetically modified animal in which the BARLP gene has been inactivated may be further included.

Here, the phrase "the BARLP gene has been inactivated" generally refers to a state in which the expression of the BARLP gene is suppressed by having a gene mutation such as insertion, deletion or substitution of a nucleotide in one or both of the paired genes of the BARLP gene. The case in which a mutated BARLP protein whose function as a normal BARLP protein is decreased or lost is expressed is also included in this "suppression of the expression of the BARLP gene". In the above-mentioned term "suppression", not only the case in which the expression of the BARLP gene is completely suppressed, but also the case in which the expression of only one gene of the paired genes of the BARLP gene is suppressed are included. In the present invention, it is preferred that the expression of the BARLP gene is specifically suppressed. Further, the site where the gene mutation is present is not particularly limited as long as it is a site to cause suppression of the expression of the gene, and for example, an exon region, a promoter region and the like can be exemplified.

In the present invention, the animal to be a subject for modification of the BARLP gene is generally a mammal other than a human, and is preferably a rodent such as a mouse, a rat, a hamster or a rabbit, and is particularly preferably a mouse among them. In the present invention, an ES cell to be a subject for modification of the BARLP gene is also preferably derived from a rodent, and is particularly preferably derived from a mouse. Incidentally, generally called "knockout animals" are also included in the genetically modified animal of the present invention.

In the nonhuman genetically modified animal and genetically modified ES cell of the present invention, as the method of artificially suppressing the expression of the BARLP gene, a method in which the whole or a part of the BARLP gene is deleted, a method in which the whole or a part of the region for regulating the expression of the BARLP gene is deleted and the like can be exemplified. However, it is preferably a method in which the BARLP gene is inactivated by inserting an exogenous gene into one or both of the paired genes of the BARLP gene. That is, in a preferred embodiment of the present invention, the genetically modified animal and genetically modified ES cell are characterized in that an exogenous gene is inserted into one or both of the paired genes of the BARLP gene.

The genetically modified animal of the present invention can be produced by a genetic engineering technique generally known to a person skilled in the art. For example, a genetically modified mouse can be produced as follows. First, DNA containing an exon region of the BARLP gene is isolated from a mouse, and an appropriate marker gene is inserted into this DNA fragment, whereby a targeting vector is constructed. This targeting vector is introduced into an ES cell line of a mouse by the electroporation method or the like, and a cell line in which homologous recombination has occurred is selected. As the marker gene to be inserted is preferably an antibiotic resistance gene such as a neomycin resistance gene. In the case where an antibiotic resistance gene is inserted, a cell line in which homologous recombination has occurred can be selected only by culturing cells with a medium containing the antibiotic. Further, in order to perform more efficient selection, it is also possible to link a thymidine kinase gene or the like to the targeting vector. By doing this, a cell line in which nonhomologous recombination has occurred can be eliminated. Further, by performing an assay for a homologous recombinant by PCR and Southern blot, a cell line in which either one of the paired genes of the BARLP gene has been inactivated can also be efficiently obtained.

In the case where a cell line in which homologous recombination has occurred is selected, there is a fear that unknown gene disruption occurs due to gene insertion occurring also at a site other than the homologous recombination site, therefore, it is preferred to produce a chimera using multiple clones. A chimeric mouse can be obtained by injection of an obtained ES cell line to the mouse blastoderm. By mating this chimeric mouse, a mouse in which either one of the paired genes of the BARLP gene has been inactivated can be obtained. Further, by mating this mouse, a mouse in which both of the paired genes of the BARLP gene have been inactivated can be obtained. Also in an animal whose ES cells have been established other than a mouse, genetic modification can be carried out by a similar method.

Subsequently, a test compound is administered to the thus produced nonhuman genetically modified animal. The administration of a test compound to the genetically modified animal can be carried out orally or parenterally.

Subsequently, the amount of food intake or body weight of the nonhuman genetically modified animal is measured. The measurement of the amount of food intake or body weight can be carried out by a method known to a person skilled in the art.

Finally, a difference in the phenotype is compared by performing comparison with a nonhuman genetically modified animal to which a test compound has not been administered. To be more specific, for example, a test compound evaluated in an in vitro evaluation system (the above-mentioned method for evaluating a compound) is administered to a nonhuman genetically modified animal and a wild type, respectively (a group without administration may be set as a control). Then, by selecting a compound with which an action of regulating eating or body weight is observed in the wild type, but is not observed in the nonhuman genetically modified animal, it can be confirmed that the test compound specifically acts on BARLP. The thus selected compound is a compound that regulates eating or body weight, and is considered to be useful as a drug for treating or preventing a disease.

Further, in the method for evaluating a compound of the present invention, by further including the above-mentioned step of evaluating a test compound using a nonhuman genetically modified animal, it becomes possible to evaluate a compound targeting BARLP in an environment close to the human body. That is, it becomes possible to observe the drug efficacy or side effects of the test compound which cannot be understood in an in vitro study, which permits more effective evaluation of a compound.

Further, as another embodiment of the method for evaluating a compound of the present invention, the method is characterized by comprising the steps of: administering a test compound to a nonhuman genetically modified animal in which a BARLP gene has been inactivated; and detecting a change in a phenotype of the nonhuman genetically modified animal caused by the administration and comparing a difference with a phenotype of a normal animal.

As the nonhuman genetically modified animal in which a BARLP gene has been inactivated, the same genetically modified animal as described in the above can be used.

As the method of administering a test compound to a nonhuman genetically modified animal in which a BARLP gene has been inactivated, oral or parenteral administration can be exemplified.

Subsequently, the phenotype of the nonhuman genetically modified animal (for example, amount of food intake or body weight) is studied. The measurement of the amount of food intake or body weight can be carried out by a method known to a person skilled in the art.

Finally, a difference in the phenotype is compared in comparison with a nonhuman genetically modified animal to which a test compound has not been administered. To be more specific, for example, a test compound evaluated in an in vitro evaluation system (the above-mentioned method for evaluating a compound) is administered to a nonhuman genetically modified animal and a wild type, respectively (a group without administration may be set as a control). Then, by selecting a compound with which an action of regulating eating or body weight is observed in the wild type, but is not observed in the nonhuman genetically modified animal, it can be confirmed that the test compound specifically acts on BARLP. The thus selected compound is a compound that regulates eating or body weight, and is considered to be useful as a drug for treating or preventing a disease.

Further, by the above-mentioned method for evaluating a compound of the present invention, evaluation of a ligand to be used in PET (positron emission tomography) can be carried out. PET is a noninvasive method for observing a biological function by radiolabeling a ligand for a substance present in the living body such as water, oxygen, glucose or an amino acid or a receptor of interest and administering the ligand to the body, and is used in research and clinical practice. PET is characterized by enabling imaging specific to a function depending on the ligand to be used as a tracer, and development of a new tracer is essential for elucidation of an unknown biological function or diagnosis of a disease.

According to the method for evaluating a compound of the present invention, by applying a PET ligand candidate substance as a test compound, it becomes possible to perform evaluation of the substance in vitro.

### (2) Substance that regulates eating or body weight

Hereinafter, the substance that regulates eating or body weight of the present invention will be described.

The substance that regulates eating or body weight of the present invention is characterized by having an action of regulating a function of BARLP. Here, the "action of regulating a function of BARLP" is not particularly limited in terms of the physiological basis of the action as long as it is an action that regulates a function of BARLP, and for example, dysfunction caused by suppression and enhancement of the expression of BARLP can be exemplified. Further, in the "action of regulating a function of BARLP", both cases of suppressing and enhancing a function of BARLP are included.

According to the finding of the present invention, an enhancement of the amount of food intake and an increase in the body weight are observed in a mouse in which BARLP is not expressed. That is, a substance that suppresses the expression or function of BARLP contributes to an increase in the body weight, therefore, it is effective as a preventive or therapeutic agent for leanness or a disease accompanied by eating disorder (for example, cibophobia, anorexia nervosa, eating disorder, hypophagia or norexia). On the other hand, a substance that enhances the expression or function of BARLP contributes to a decrease in the body weight, therefore, it is effective as a preventive or therapeutic agent for obesity or a disease accompanied by obesity (for example, obesity, diabetes, abnormal hormone secretion, gout, fatty liver, hypercholesterolemia, hyperlipidemia, arteriosclerosis or glaucoma).

The substance that suppresses or enhances the expression of BARLP is not particularly limited, and examples thereof include single compounds such as natural compounds, organic compounds, inorganic compounds, proteins and peptides, and antibodies, antisenses, RNAi and ribozymes of BARLP.

In the case where the substance that regulates eating or body weight of the present invention (a compound selected by the method for evaluating a compound of the present invention described above is included) is used as a drug for humans or other animals, it is possible to administer the substance by formulating it into a preparation by a known pharmaceutical method other than the direct administration of the substance per se to a patient. For example, it can be used orally as a tablet, if necessary coated with a sugar, a capsule, an elixir or a microcapsule, or parenterally in the form of an injection of a sterile solution or a suspension with water or a pharmaceutically acceptable liquid other than water. For example, it is considered that the substance is formulated into a preparation by appropriately combining a pharmacologically acceptable carrier or medium, specifically sterile water, physiological saline, a vegetable oil, an emulsifier, a suspending agent, a surfactant, a stabilizer, a flavoring agent, an excipient, a vehicle, a preservative or a binder, and mixing them in a unit dosage form as required by generally admitted pharmaceutical practice.

Examples of an additive which can be mixed in a tablet or a capsule include binders such as gelatin, cornstarch, tragacanth gum and gum arabic; excipients such as crystalline cellulose; swelling agents such as cornstarch, gelatin and alginic acid; lubricants such as magnesium stearate; sweeteners such as sucrose, lactose and saccharine; and flavoring agents such as peppermint, acamono oil and cherry. In the case where the preparation unit is in the capsule form, a liquid carrier such as fat and oil may further be added to the above-mentioned materials. A sterile composition for injection can be formulated according to usual pharmaceutical practice using a vehicle such as distilled water for injection.

Examples of an aqueous solution for injection include physiological saline and isotonic solutions containing glucose or other adjuvants (for example, D-sorbitol, D-mannose, D-mannitol and sodium chloride), and may be used in combination with an appropriate solubilizing agent such as an alcohol, specifically ethanol, a polyalcohol such as propylene glycol or polyethylene glycol or a nonionic surfactant such as Polysolvate 80 (TM) or HCO-50.

Examples of an oily liquid include sesame oil and soybean oil, and may be used in combination with a solubilizing agent such as benzyl benzoate or benzyl alcohol. In addition, it may be further mixed with a buffer such as a phosphate buffer or a sodium acetate buffer, a soothing agent such as procaine hydrochloride, a stabilizer such as benzyl alcohol or phenol, or an antioxidant. The thus prepared injection is usually filled into an appropriate ampoule.

The administration thereof to a patient can be carried out by a method known to a person skilled in the art, for example, by intraarterial injection, intravenous injection, subcutaneous injection or the like, and also by intranasal, transbronchial, intramuscular, percutaneous or oral administration. The dose varies depending on the body weight or age of a patient, an administration route or the like, however, a person skilled in the art can appropriately select a suitable dose. Further, if the compound can be encoded by DNA, it is also possible that the DNA is inserted into a vector for gene therapy and gene therapy is carried out. The dose and administration route vary depending on the body weight, age or symptoms of a patient or the like, however, a person skilled in the art can appropriately select them.

The dose of the compound varies depending on the symptoms, however, in the case of oral administration, the dose thereof is considered to be about 0.1 to 100 mg per day, preferably about 1.0 to 50 mg per day, more preferably about 1.0 to 20 mg per day for an adult (assuming of 60 kg of body weight).

In the case of parenteral administration, a single dose thereof varies depending on the subject to be administered, target organ, symptoms, or administration route. However, in the case of, for example, an injection for generally an adult (assuming of 60 kg of body weight), it is considered to be convenient to administer the compound in an amount of generally about 0.01 to 30 mg per day, preferably about 0.1 to 20 mg per day, more preferably about 0.1 to about 10 mg per day by intravenous injection.

### (Examples)

Hereinafter, the present invention will be described further specifically with reference to Examples, however, the present invention is not limited to the following Examples.

### Test Example 1

### (BARLP (-/-) mice)

BARLP (-/-) mice were produced by crossing hetero BARLP (+/-) (Deltagen) produced by homologous recombination. The genotype was confirmed by PCR using mice in the weaning period.

### Example 1

### (Study of body weight of BARLP (-/-) mice)

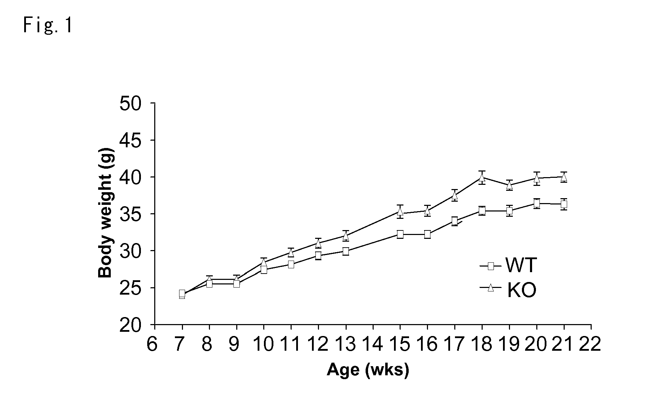

In the following test, BARLP (-/-) and wild type (WT) at 7 weeks of age were used. During rearing the mice, water and pellet food (CE-2: Nippon CLEA) were given ad libitum. Further, the mice to be used in the test were reared in an individual cage under conditions of room temperature of 23 ± 2°C, a humidity of 55 ± 15% with a 12-hour light and dark cycle. The body weight was observed over 14 weeks. As shown in Fig. 1, BARLP (-/-) mice showed a significant increase in the body weight in comparison of BARLP (-/-) with WT.

### Example 2

### (Study of amount of food intake of BARLP (-/-) mice)

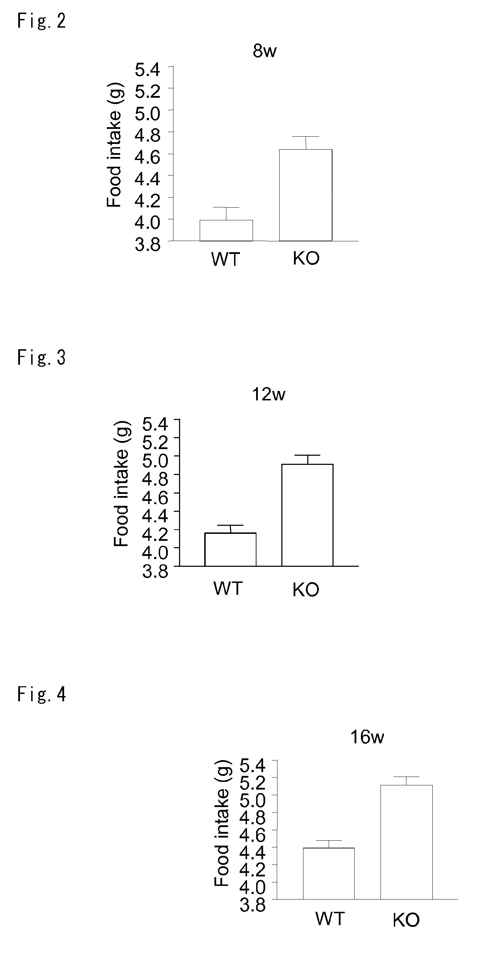

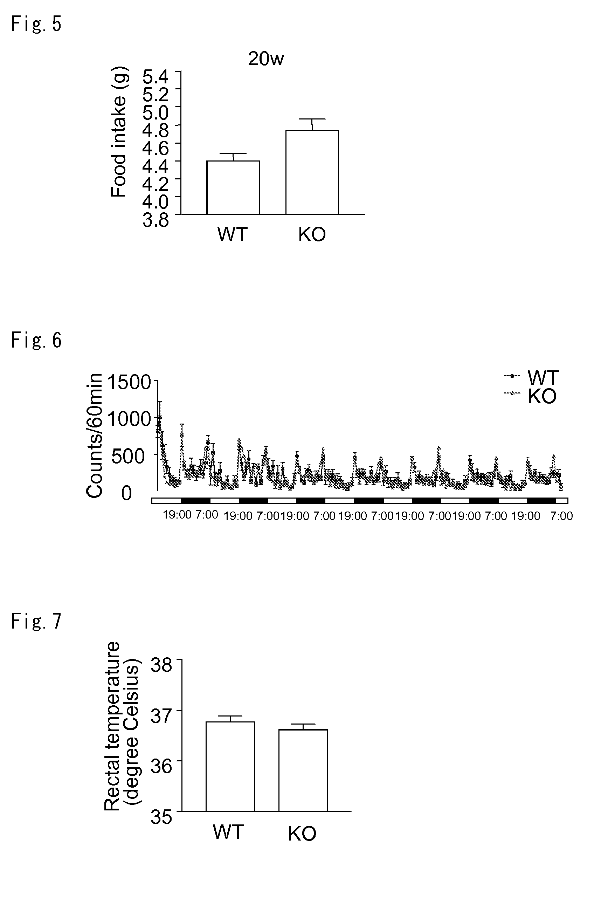

Further, with regard to the amount of food intake, average amounts of food intake per day were calculated based on the amounts of food intake for 4 days at 8 weeks of age (Fig. 2), at 12 weeks of age (Fig. 3), at 16 weeks of age (Fig. 4) and at 20 weeks of age (Fig. 5), respectively. As shown in Figs. 2 to 5, it could be confirmed that the amount of food intake of BARLP (-/-) mice is significantly larger than that of WT.

### Example 3

### (Study of locomotor activity of BARLP (-/-) mice)

The locomotor activity of BARLP (-/-) mice was measured with an activity monitoring system (NS-AS01: Neuroscience) using mice at 18 weeks of age. At this time, the locomotor activity was monitored from the top of 24 test cages with infrared sensors, respectively. Further, during the test, water and food were given ad libitum to all mice in the cages. With regard to the locomotor activity, data summarized every 60 minutes was analyzed with a test animal locomotor activity monitoring system (AB System-24A: Neuroscience). The monitoring of locomotor activity was carried out for 7 days. As shown in Fig. 6, there is no significant difference in the locomotor activity between BARLP (-/-) and WT mice, and it could be confirmed that there is no relationship between an increase in the body weight or amount of food intake in the BARLP (-/-) mice shown in Examples 1 and 2 and the motor activity.

### Example 4

### (Study of rectal temperature of BARLP (-/-) mice)

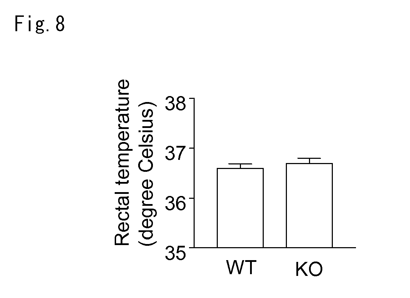

The rectal temperature was measured with a digital thermometer (BAT-12: Physitemp Instruments) with a rectal probe from 9 o'clock to 11 o'clock in the morning. The rectal probe was inserted into the anus to a depth of 2 cm. The rectal temperature was measured for mice at 17 and 19 weeks of age. As shown in Fig. 7 (17 weeks of age) and Fig. 8 (19 weeks of age), there is no significant difference in the rectal temperature between BARLP (-/-) and WT mice, and it could be confirmed that there is no relationship between an increase in the body weight or amount of food intake in the BARLP (-/-) mice shown in Examples 1 and 2 and the body temperature.

### Industrial Applicability

There is a certain relationship between the expression level of BARLP and the body weight or amount of food intake, and it becomes possible to evaluate a compound targeting BARLP based on the relationship. Further, it becomes possible to provide a ligand (for example, a substance that regulates body weight) for BARLP obtained by the evaluation.

With the use of such evaluation and substance, a preventive or therapeutic agent for leanness or a disease accompanied by eating disorder (for example, cibophobia, anorexia nervosa, eating disorder, hypophagia or norexia) is provided. Further, a substance that enhances the expression of BARLP contributes to a decrease in the body weight, therefore, a preventive or therapeutic agent for obesity or a disease accompanied by obesity (for example, obesity, diabetes, abnormal hormone secretion, gout, fatty liver, hypercholesterolemia, hyperlipidemia, arteriosclerosis or glaucoma) is provided.

## Claims

1. A method for evaluating a compound that regulates eating or body weight, **characterized by** comprising the steps of:
preparing a cell expressing BARLP by introducing a BARLP gene;
bringing a test compound into contact with the cell; and
detecting a specific binding of the test compound to the BARLP.

2. A method for evaluating a compound that regulates eating or body weight, **characterized by** comprising the steps of:
preparing a cell expressing BARLP by introducing a BARLP gene;
bringing a test compound into contact with the cell;
measuring the activity of an intracellular signal transducer induced by the contact; and
comparing said activity with the activity of the intracellular signal transducer in the absence of contact with the test compound.

3. A method for evaluating a compound that regulates eating or body weight, **characterized by** comprising the steps of:
preparing a cell expressing BARLP by introducing a BARLP gene;
bringing a test compound into contact with the cell;
measuring the expression level of the BARLP or an intracellular signal transducer mediated by the BARLP; and
selecting the test compound which increased or decreased the expression level of the BARLP in comparison with the case in the absence of contact with the test compound.

4. The method for evaluating a compound according to any one of claims 1 to 3, **characterized by** further comprising the step of evaluating a test compound using a nonhuman genetically modified animal in which the BARLP gene has been inactivated.

5. A method for evaluating a compound that regulates eating or body weight, **characterized by** comprising the steps of:
administering a test compound to a nonhuman genetically modified animal in which a BARLP gene has been inactivated; and
detecting a change in a phenotype of the nonhuman genetically modified animal caused by the administration and comparing a difference with a phenotype of a normal animal.

6. A method for evaluating a compound that regulates eating or body weight, **characterized by** comprising the steps of:
bringing a test compound into contact with BARLP; and
detecting a change in the activity of BARLP caused by the contact.

7. A BARLP ligand, **characterized in that** it is isolated by the method for evaluating a compound according to any one of claims 1 to 6.

8. A substance that regulates eating or body weight, **characterized by** having an action of regulating a function of BARLP.

9. The substance according to claim 8, **characterized in that** the substance that regulates eating or body weight is one member selected from the group consisting of natural compounds, organic compounds, inorganic compounds, proteins, peptides, antibodies, antisenses, RNAi and ribozymes.
